(19) Europäisches Patentamt
European Patent Office
Office européen des brevets

(11) **EP 3 120 839 B1**

(12) **EUROPEAN PATENT SPECIFICATION**

(45) Date of publication and mention
of the grant of the patent:
**19.12.2018 Bulletin 2018/51**

(51) Int Cl.:
***A61K 9/14*** (2006.01)   ***A61K 9/20*** (2006.01)
***A61K 31/7048*** (2006.01)

(21) Application number: **15178009.5**

(22) Date of filing: **23.07.2015**

(54) **NON-MECHANICAL PROCESS FOR THE MICRONIZATION OF DIGOXIN**

NICHTMECHANISCHES VERFAHREN ZUR MIKRONISIERUNG VON DIGOXIN

PROCÉDÉ NON MÉCANIQUE POUR LA MICRONISATION DE DIGOXINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**25.01.2017 Bulletin 2017/04**

(73) Proprietor: **Mehta, Raman
Kowloon (CN)**

(72) Inventor: **Mehta, Raman
Kowloon (CN)**

(74) Representative: **Zardi, Marco
M. Zardi & Co. SA
Via Pioda 6
6900 Lugano (CH)**

(56) References cited:
**US-A- 5 062 959    US-A1- 2004 048 809**

• **M. DRAGUET-BRUGHMANS ET AL: "The
physicochemical properties of digoxin",
JOURNAL OF PHARMACEUTICAL AND
BIOMEDICAL ANALYSIS, vol. 3, no. 3, 1 January
1985 (1985-01-01) , pages 227-234, XP055239391,
US ISSN: 0731-7085, DOI:
10.1016/0731-7085(85)80027-3**

EP 3 120 839 B1

## Description

## FIELD OF INVENTION

The present invention relates to the field of micronized drugs. A new method is described for obtaining digoxin in a micronized form, resulting in highly homogeneous powder, compliant with the current regulatory requirements for digoxin and characterized by an enhanced stability of the active principle.

## BACKGROUND OF THE INVENTION

Digoxin is a cardioactive glycoside naturally present in the leaves of *Digitalis purpurea* and *Digitalis lanata lanata (foxglove).* These plants were known and used for long time in traditional medicine, with first written reports of their use dating back to the end of the eighteenth century. The active glycoside was subsequently isolated, characterized and used as active principle in a variety of medicaments. Today, despite the rise in synthetic and semi-synthetic drugs, digoxin still remains a fundamental therapeutic principle, being cited in the World Health Organization's List of Essential Medicines.

Digoxin has a general tonic effect on cardiac muscle increasing its force of contraction and tone. It is used in therapy in the treatment of various heart conditions, in particular atrial fibrillation and atrial flutter: by slowing down the conduction in the AV node and increasing its refractory period, digoxin can reduce the ventricular rate. Digoxin is also used in the treatment of heart failure, especially in patients unresponsive to diuretic and ACE inhibitor treatment.

Digoxin is preferably administered orally. It has a narrow therapeutic window and for these reasons the control of its release is of great importance. In fact, a very rapid release may result in excessively high plasma levels and therefore in a number of side effects; if the release is too slow incomplete absorption occurs, and no therapeutic effect is obtained.

Many literature reports and patents disclose compositions of digoxin with excipients for gradual and controlled release, making it compliant with the required pharmaceutical standards (for example the United States Pharmacopoeia sets a specification for digoxin requiring that at least 85% digoxin be released in 60 min). An important parameter responsible for the solubility and, indirectly, for the bioavailablity of digoxin, is the drug particle size. In particular, substances like digoxin, being poorly soluble in aqueous environment, might benefit from a reduction in particle size and consequent increase of specific surface; these interrelations are summarized in the following equation, where dM/dt is the dissolution rate, A the specific surface area of the drug particle, D the diffusion coefficient, h the diffusion layer thicknes $C_s$ the saturation solubility, and $C_t$ the drug concentration at time t.

$$\frac{dM}{dt} = \frac{AD\,(Cs - Ct)}{h}$$

It will be noted that, since the surface area increases with decreasing particle size, a higher dissolution rate may be achieved through the reduction of the particle size of the drug substance.

In the case of digoxin, micronization to a particle size of below 50 micrometers is particularly appreciated, since it easily allows to process it in formulations with a delivery time compliant with the above referred pharmaceutical standards for digoxin; an example thereof is disclosed in US2004/0048809. The patent US 5 062 959 describes a process to purify/enrich heart glycosides, and exemplifies inter alia a process of crystallization of purified digoxin.

In pharmaceutical technology, particle size reduction can be achieved via micronization processes. These are typically mechanical process, performed in dry conditions, whereby a macro-sized raw material undergoes high-impact mechanical treatment, e.g. via milling, grinding, etc; these techniques are based on friction to reduce particle size. However, the standard processes of micronization are not fully satisfactory, especially when used with highly active principles requiring a precise micronization profile: in particular, the degree of homogeneity in particle size may vary from different drug lots, and this reflects into undesired variations in bioavailability and activity. Moreover digoxin is very labile to mechanical treatments: these were occasionally reported to cause the formation of degradation products, made evident by a colour change of the powder after micronization; the partial degradation of digoxin, is further responsible for an undesired reduction of activity and/or to toxicity due to degradation products.

A first object of the present invention is to develop a new, non-mechanical process of digoxin micronization. A further object is to make available a new process for obtaining digoxin in a micronized, highly homogeneous form. A further object is to make available a new micronization process for digoxin, having a low impact on the chemical/physical features of this active principle. Also disclosed is digoxin which, once formulated for oral administration, meets the current release standards for digoxin, while not being exposed to degradation. Also disclosed is a stabilized form of micronized digoxin. Also disclosed is a micronized digoxin which is highly pure (i.e. exempt or substantially exempt) from digoxin degradation products. These and further objectives are obtained by the invention described below.

## SUMMARY OF THE INVENTION

The present invention discloses a new, non-mechanical process of micronization, capable to reduce the particle size of digoxin from ordinary level to a selected micrometer range. Micronization is obtained via a specific treatment, to be performed on a purified and concentrated digoxin solution in an organic solvent. The purified and concentrated digoxin solution is obtainable via a sequence of solvent treatments; then, after reaching the required concentration, the solution is further concentrated, obtaining the precipitation of digoxin; the reaction mixture is then added with methanol under stirring for a suitable time. The recovered precipitate consists in a digoxin with particle size comprised between 20 and 30 micrometers (for at least 90% by weight of the obtained particles), and exempt from degradation products.

## DETAILED DESCRIPTION OF THE INVENTION

In the expression "concentrated and purified digoxin solution", the term "concentrated" means a concentration of digoxin between 50 and 200 g/ L, preferably 70-150 g/L, most preferably 100-120 g/L.

In the expression "concentrated and purified digoxin solution", the term "purified" means a digoxin with purity of 90.0 - 99.9%, preferably 95.0 - 99.9%, most preferably between 98.0 and 99.9% (calculated on the digoxin in dry form).

A preferred, although non-limitative, procedure to obtain the purified concentrated digoxin solution suitable for the present process is the following.

a) Crude digoxin, as normally available by known industrial methods, is solubilised with a hydroalcoholic solvent (preferably butanol:water) in presence of a base (preferably sodium carbonate), followed by heating for a suitable time (preferably at least 90°C for 5-15 hours). The solution is then cooled (typically at 5-15°C for a 5-15 hours). The precipitate is filtered and dried.

b) The precipitate obtained in a) is dissolved in an organic solvent mixture (preferably methanol:methylene chloride), then reprecipitated by addition of water under stirring (preferably at 15-25°C for 3-7 hours); the precipitate is filtered and dried.

c) The precipitate obtained in b) is dissolved in a organic solvent mixture (preferably methanol:methylene chloride), added with carbon and filtered; the filtered solution is passed through one or more beds of alumina, and then concentrated, obtaining a purified concentrated digoxin solution. This solution has a concentration typically comprised comprised between 50 and 200 g/ L, preferably 70-150 g/L.

In accordance with the present invention, the purified and concentrated digoxin solution is further concentrated until complete precipitation of the product (i.e. until no further precipitation is observed). Concentration is suitably obtained under vacuum, at room temperature, and in stirring conditions; once precipitation is complete, the solution is added with methanol (preferably 1-2 volumes, more preferably 1.5 volumes per volume of said further concentrated solution) and stirred at room temperature for a period of 1-6 hours, preferably 2-5 hours, most preferably 3-4 hours; the precipitate is then filtered, washed with a polar solvent, preferably acetone, and dried. Drying can be performed e.g. under vacuum, at 20-40°C for 24 hrs, however the drying conditions can be easily varied and adapted in function of the scale of operation.

The digoxin thus obtained has a high purity and is ready to be incorporated in the usual pharmaceutical formulations, typically tablets; it has particle size of 20-30 micrometers (90% of the particles) and is exempt from degradation products, as evident by a wholly neutral colour, and further confirmable analytically.

The obtained digoxin can be formulated with common excipients. Suitable excipients are cellulose products like microcrystalline cellulose, cellulose ethers like methylcellulose,ethylcellulose, hydroxyethylcellulose, crosslinked polymers (e.g. polyplasdone XL, hyaluronic acid or alginic acid cross-linked with urea), gums from plants, etc. Common diluents like mannitol, fructose, as well as compression aids like magnesium stearate, can also be used. These formulations can be prepared in accordance with conventional methods, like those described in "Remington's Pharmaceutical Handboock" Mack Publishing Co., N.Y. USA, together with suitable excipients. The so-formulated digoxin is suitable to obtain a release rate compliant with the current pharmaceutical standards for digoxin, dissolving at a sufficiently fast rate, while avoiding excessive plasma peaks.

The invention is now described by reference to the following non-limitative examples.

## Example 1

Crude digoxin (1,5 Kg) obtained by industrial traditional methods (digoxin content 85%) was dissolved in 3 times butanol/water mixture (88:12) (4,5 L) . 30,0 g solid sodium carbonate were added and the mixture was refluxed (95±2°C) for 10 hrs. The reaction mass was then cooled to 10°C and then maintained for 10 hrs at temperature below 10°C. The material was filtered and dried under vacuum at 70°C for 12 hrs. The material so obtained was dissolved in 30 L of a mixtrure methylene dichloride: methanol (1:1 v/v). After complete dissolution, further 30 L of methylene dichloride were added, followed by slow addition of 30 L of water. The reaction mass was stirred up to 5 hrs at 18-20°C, then filtered and dried under vacuum at 70°C for 12 hrs, obtaining 1.2 Kgs of dried material. The dried material was then dissolved in 20-24 L of a mixture methylene dichloride:methanol (1:1 v/v) at 35-40°C. The mixture was then added with 0.4-0.6 Kg carbon and stirred for 30 minutes at 35-40°C. It was then filtered through Hyflo (diatomaceous earth) bed and the filtered solution was further passed through Alumina (1.5 Kg), Hyflo and Alumina beds; the beds were washed with 6 L of methylene dichloride:methanol (1:1 v/v). The combined filtrates were concentrated up to 10 L without vacuum. The resulting solution was further concentrated under vacuum and stirring up to 4 L, observing the formation of a precipitate; once the precipitation was complete, the solution was added with 6 L of methanol under stirring for 3-4 hours; the precipitate was finally filtered, washed with acetone and dried under vacuum at 25-40°C for 24 h; digoxin (0.95 Kg) with particle size 20-30 micrometers (> 90% of the particles) was obtained.

**Reference Example 2**

This example describes an embodiment of the preparation of a cardiotonic composition (tablet) based on the micronized digoxin according to example 1.

| | |
|---|---|
| Micronized digoxin | 0,200 mg |
| Microcristalline cellulose | 100 mg |
| Mannitol | 20 mg |
| Fructose | 40 mg |
| Polyplasdone XL | 8 mg |
| Magnesium stearate | 4 mg |

**Claims**

1. A process to obtain a non-mechanically micronized digoxin, wherein a purified and concentrated solution of digoxin in an organic solvent, having a digoxin concentration between 50 and 200 g/L, is further concentrated until obtaining digoxin precipitation, then added with methanol, stirred for 1-6 hours, followed by recovery and drying of the precipitate.

2. The process of claim 1, wherein the obtained micronized digoxin is exempt from degradation products.

3. The process of claims 1-2, wherein said digoxin concentration is comprised between 70 and 150 g/L.

4. The process of claims 1-3, wherein said organic solvent is a mixture of a chlorinated solvent with an alcohol.

5. The process of claims 1-4, wherein said organic solvent is mixture of methylene dichloride with methanol.

6. The process of claims 1-5, wherein said organic solvent is a 1:1 v/v mixture of methylene dichloride with methanol.

7. The process of claims 1-6, wherein said added methanol is used in an amount of 1-2 volumes per volume of said concentrated solution.

8. The process of claims 1-7, wherein said added methanol is used in an amount of 1.5 volumes per volume of said concentrated solution.

9. The process of claims 1-8 wherein the solution added with methanol is stirred for 3-4 hours.

10. The process of claims 1-9, wherein said further concentration is performed under vacuum, at room temperature.

11. The process of claims 1-10, wherein the said purified concentrated digoxin has been prepared by the following steps:

   i) crude digoxin, is solubilised in a hydroalcoholic solvent in presence of a base, heated to at least 90°C, followed by cooling of the solution and recovery of the obtained precipitate.
   ii) the precipitate obtained in a) is dissolved in a solvent mixture, then precipitated by addition of water under stirring, followed by recovery of the precipitate.
   iii) the precipitate obtained in b) is dissolved in an organic solvent mixture, added with carbon and filtered; the filtered solution is passed through one or more filtering beds and is then concentrated to a value comprised between 30-150 g/L.

**Patentansprüche**

1. Verfahren zum Erhalten eines nichtmechanisch mikronisierten Digoxins, bei dem eine gereinigte und konzentrierte Digoxinlösung in einem organischen Lösungsmittel, die eine Digoxinkonzentration zwischen 50 und 200 g/L hat, weiter konzentriert wird, bis die Digoxinausfällung erhalten wird, dann mit Methanol versetzt wird, 1-6 Stunden lang gerührt wird, gefolgt von Gewinnung und Trocknen der Ausfällung.

2. Verfahren nach Anspruch 1, bei dem das erhaltene mikronisierte Digoxin frei von Abbauprodukten ist.

3. Verfahren nach den Ansprüchen 1-2, bei dem die Digoxinkonzentration zwischen 70 und 150 g/L beträgt.

4. Verfahren nach den Ansprüchen 1-3, bei dem das organische Lösungsmittel eine Mischung von einem chlorierten Lösungsmittel mit einem Alkohol ist.

5. Verfahren nach den Ansprüchen 1-4, bei dem das organische Lösungsmittel eine Mischung von Methylendichlorid mit Methanol ist.

6. Verfahren nach den Ansprüchen 1-5, bei dem das organische Lösungsmittel eine 1:1 v / v-Mischung von Methylendichlorid mit Methanol ist.

**7.** Verfahren nach den Ansprüchen 1-6, bei dem das hinzugefügte Methanol in einer Menge von 1-2 Volumina pro Volumen der konzentrierten Lösung verwendet wird.

**8.** Verfahren nach den Ansprüchen 1-7, bei dem das hinzugefügte Methanol in einer Menge von 1,5 Volumina pro Volumen der konzentrierten Lösung verwendet wird.

**9.** Verfahren nach den Ansprüchen 1-8, bei dem die mit Methanol versetzte Lösung 3-4 Stunden lang gerührt wird.

**10.** Verfahren nach den Ansprüchen 1-9, bei dem die weitere Konzentrierung unter Vakuum bei Raumtemperatur durchgeführt wird.

**11.** Verfahren nach den Ansprüchen 1-10, bei dem das gereinigte konzentrierte Digoxin durch die folgenden Schritte zubereitet wurde:

i) Rohdigoxin wird in einem wässrig-alkoholischen Lösungsmittel im Beisein einer Base gelöst, auf mindestens 90°C erhitzt, gefolgt von Kühlung der Lösung und Gewinnung der erhaltenen Ausfällung.
ii) die in a) erhaltene Ausfällung wird in einer Lösungsmittelmischung gelöst, dann durch Hinzufügung von Wasser unter Rühren ausgefällt, gefolgt von Gewinnung der Ausfällung.
iii) die in b) erhaltene Ausfällung wird in einer organischen Lösungsmittelmischung gelöst, mit Kohlenstoff versetzt und gefiltert; die gefilterte Lösung wird durch ein oder mehrere Filtrierbetten hindurchgeführt und wird dann auf einen Wert konzentriert, der zwischen 30 -150 g/L beträgt.

**Revendications**

**1.** Procédé d'obtention d'une digoxine micronisée de manière non mécanique, dans lequel une solution purifiée et concentrée de digoxine dans un solvant organique, présentant une concentration en digoxine comprise entre 50 g/l et 200 g/l, est davantage concentrée jusqu'à obtention d'un précipité de digoxine, du méthanol est ensuite ajouté, le mélange est soumis à agitation pendant 1 à 6 heures, puis le précipité est récupéré et séché.

**2.** Procédé selon la revendication 1, dans lequel la digoxine micronisée obtenue est exempte de produits de dégradation.

**3.** Procédé selon les revendications 1 et 2, dans lequel ladite concentration en digoxine est comprise entre 70 g/l et 150 g/l.

**4.** Procédé selon les revendications 1 à 3, dans lequel ledit solvant organique est un mélange d'un solvant chloré avec un alcool.

**5.** Procédé selon les revendications 1 à 4, dans lequel ledit solvant organique est un mélange de dichlorure de méthylène avec du méthanol.

**6.** Procédé selon les revendications 1 à 5, dans lequel ledit solvant organique est un mélange à 1:1 v/v de dichlorure de méthylène avec du méthanol.

**7.** Procédé selon les revendications 1 à 6, dans lequel ledit méthanol ajouté est utilisé selon une proportion de 1 à 2 volumes par volume de ladite solution concentrée.

**8.** Procédé selon les revendications 1 à 7, dans lequel ledit méthanol ajouté est utilisé selon une proportion de 1,5 volumes par volume de ladite solution concentrée.

**9.** Procédé selon les revendications 1 à 8, dans lequel la solution obtenue après ajout du méthanol est soumise à agitation pendant 3 à 4 heures.

**10.** Procédé selon les revendications 1 à 9, dans lequel ladite concentration supplémentaire est réalisée sous vide à température ambiante.

**11.** Procédé selon les revendications 1 à 10, dans lequel ladite digoxine concentrée purifiée a été préparée en suivant les étapes suivantes :

i) de la digoxine brute est solubilisée dans un solvant hydro-alcoolique en présence d'une base, chauffée à au moins 90 °C, puis la solution est refroidie et le précipité obtenu est récupéré.
ii) le précipité obtenu en a) est dissous dans un mélange de solvant, puis précipité par ajout d'eau sous agitation, puis le précipité est récupéré.
iii) le précipité obtenu en b) est dissous dans un mélange de solvant organique, se voit ajouter du carbone puis est filtré ; la solution filtrée est passée sur un ou plusieurs lits de filtration puis est ensuite concentrée à une valeur comprise entre 30 g/l et 150 g/l.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040048809 A
- US 5062959 A

**Non-patent literature cited in the description**

- Remington's Pharmaceutical Handboock. Mack Publishing Co,